(19) Europäisches Patentamt
European Patent Office
Office européen des brevets

(11) **EP 3 296 736 B1**

(12) **EUROPEAN PATENT SPECIFICATION**

(45) Date of publication and mention
of the grant of the patent:
**22.05.2019 Bulletin 2019/21**

(21) Application number: **16189761.6**

(22) Date of filing: **20.09.2016**

(51) Int Cl.:
*G01N 33/22* *(2006.01)*          *G01N 25/18* *(2006.01)*
*G01N 29/024* *(2006.01)*        *G01S 7/41* *(2006.01)*
*G01N 22/00* *(2006.01)*

(54) **METHOD AND SYSTEM FOR MEASURING THE ENERGY CONTENT OF GAS**

VERFAHREN UND SYSTEM ZUR MESSUNG DES ENERGIEGEHALTS VON GAS

PROCÉDÉ ET SYSTÈME POUR MESURER LA TENEUR ÉNERGÉTIQUE DU GAZ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**21.03.2018 Bulletin 2018/12**

(73) Proprietor: **SP Technical Research Institute Of
Sweden
50115 Borås (SE)**

(72) Inventor: **Büker, Oliver
SE-501 15 Borås (SE)**

(74) Representative: **Finnegan Europe LLP
1 London Bridge
London SE1 9BG (GB)**

(56) References cited:
**WO-A1-00/43806     US-B1- 6 244 097**

- **JAESCHKE M ET AL: "THERMODYNAMIC
RESEARCH IMPROVES ENERGY
MEASUREMENT IN NATURAL GAS",
INTERNATIONAL JOURNAL OF
THERMOPHYSICS, NEW YORK, US, US, vol. 23,
no. 4, 1 July 2002 (2002-07-01), pages 1013-1031,
XP008017844, DOI: 10.1023/A:1016385902427**

**Description**

Field of the Invention

This invention relates to methods and systems for measuring the energy content of a gas, such as a hydrocarbon fuel gas flowing in a pipeline.

A known system disclosed in US6,244,097 determines the energy content of a gas using the correlation of the speed of sound in the gas with its dielectric constant and the carbon dioxide content of the gas.

Background

The energy content of fuel gases such as natural gas (NG), liquefied natural gas (LNG), compressed natural gas (CNG), biogas, etc. can vary as a result of variations in the composition of the gases, both in terms of hydrocarbon mix, and in terms of the content of calorifically-inert gases such as nitrogen and carbon dioxide. Customers for fuel gases pay according to the energy of the delivered gas. This means it is necessary to determine the mass or volume and the energy content of the gas (expressed as energy per unit mass or unit volume) delivered so that the total energy delivery can be determined. Consequently, a simple (mass or volumetric) flow measurement will only provide the delivered mass or volume and not an accurate value for the energy content of the gas delivered through a pipeline. Such measurement is sometimes known as "fiscal" monitoring or measurement, for example fiscal metering used for custody transfer.

The energy content or calorific value (the term "energy content" used herein includes calorific value and heat value) of gases can be determined calorimetrically, but it has been difficult to provide such measurements on-line for flowing gases. Alternatively, the chemical composition of the gases can be determined by gas chromatography (GC). This allows the energy content to be calculated. However, again such systems have been difficult to provide as an on-line, (quasi) real time measurement and the instruments are complex and expensive.

In order to avoid the problems of full composition analysis, or direct calorimetry, a number of techniques known as "correlation" measurement have been proposed. In correlation measurements, a series of "incomplete" measurement values are obtained, such as physical properties or measurements of specific components, and correlations developed that indicate the energy content of the gas providing such values. The accuracy of such techniques depends on the parameters selected for measurement and the techniques used to measure the parameters. While correlation measurements can provide reasonable accuracy, the complexity of some of the measurement technologies, and their costs have meant that adoption has been limited to date.

This invention aim to provide a correlation measurement technique that is based on robust and widely available technologies with the aim of reducing lifecycle costs.

Summary

A first aspect of the invention provides a method of measuring the energy content of a gas, comprising: measuring the speed of sound in the gas; measuring the relative permittivity (dielectric constant) of the gas using a guided wave radar sensor; and correlating the measured speed of sound and relative permittivity to derive the energy content of the gas.

Where the gas is flowing in a conduit, the measurements of speed of sound and relative permittivity are made on the flowing gas. The measurement can further comprise determining volume, pressure, and temperature parameters of the flowing gas, and using the parameters to derive the energy content of the gas.

Measuring the relative permittivity of the gas can comprise a time domain reflectometry measurement of a microwave pulse in a wave guide.

Guided wave radar sensors are widely available as level sensors for process monitoring, typically being used to measure the level of a liquid or solid in a container or process line. The time of flight measurement in a guided wave radar sensor is dependent on the relative permittivity of the medium though which the wave propagates.

The method can also comprise measuring the carbon dioxide content of the gas, and correlating the measured speed of sound, relative permittivity, and carbon dioxide content to derive the energy content of the gas.

The gas can be natural gas (NG), liquefied natural gas (LNG), compressed natural gas (CNG) or biogas. Where the gas is LNG, it may not be necessary to measure carbon dioxide content. The term "gas" used herein includes not only hydrocarbons in the gas phase, such as might be used for gas fuel, but also the liquefied forms, such as LNG, typically used for storage and transportation.

A second aspect of the invention provides a system for measuring the energy content of a gas, comprising a first sensor for measuring the speed of sound in the gas; a second sensor for measuring the relative permittivity of the gas using a guided wave radar sensor; and a correlator configured to correlate the measured speed of sound and relative permittivity to derive the energy content of the gas.

The system can further comprise a conduit housing the sensors and through which the gas flows. The system can also comprise further sensors for determining volume, pressure, and temperature parameters of the flowing gas, wherein the correlator is configured to use the parameters to derive the energy content of the gas.

The first sensor can comprise an ultrasonic flow meter or another sensor which is capable to measure the speed of sound

The second sensor can comprise a microwave source and a waveguide of predetermined configuration, the second sensor being configured to provide a time domain reflectometry measurement of a microwave pulse reflected by a predetermined feature of the waveguide configuration, such as the end of a reference rod mounted on the waveguide, for determining the relative permittivity of the gas.

The system can also comprise a third sensor, such as an infrared sensor, for measuring the carbon dioxide content of the gas, and wherein the correlator is configured to use the measured speed of sound, relative permittivity (dielectric constant) and carbon dioxide content to derive the energy content of the gas.

The system can also comprise a processor configured to receive the output of the correlator as an input, and to output values of superior calorific value, Wobbe index and normal density of the gas.

The speed of sound measurement may be replaced by a density measurement in certain cases. The system may also comprise additional sensors to provide multiple measurements of a given parameter, or measurements of further parameters depending on requirements.

Other aspects of the invention will be apparent from the following description.

Drawings

Figure 1 shows a schematic view of a guided wave radar level measurement system;
Figure 2 shows a plot of levels in methane obtained with a system corresponding to Figure 1;
Figure 3 shows a plot of measured and theoretical dielectric constant with respect to pressure for nitrogen and methane;
Figure 4 shows a schematic view of a system for determining the energy content of a gas.

Description

Various techniques for correlation measurements to determine the energy content of gases have been proposed previously. Examples can be found in "Thermodynamic research improves energy measurement of natural gas" M. Jaeschke, Thermochimica Acta 382 (2002) 37 - 45, and in US 627380 and US 2002/0124630. Among the techniques disclosed, measurements of relative permittivity or dielectric constant ($\varepsilon_r$) are disclosed. A re-entrant cavity device operated as an LC resonator is disclosed for deriving relative permittivity values. Such devices have high production costs and technical tolerances and so have not been widely adopted for such use.

This invention is based on the recognition that measurements made using guided wave radar (GWR) devices can provide the necessary information for the relative permittivity of a gas phase to be determined with sufficient accuracy for energy content monitoring.

In recent years GWR devices have become a popular method of measuring levels such as liquid levels in process equipment, for example within the oil and gas industry. GWR level sensors are considered to offer reliability and maintenance advantages over many other measurement technologies that have traditionally been used. In general GWR level sensors are seen as being unaffected by the properties of the gas present above the liquid at many process conditions. This is in fact only partially true and elevated gas densities can lead to a loss of accuracy when using GWR for level measurement.

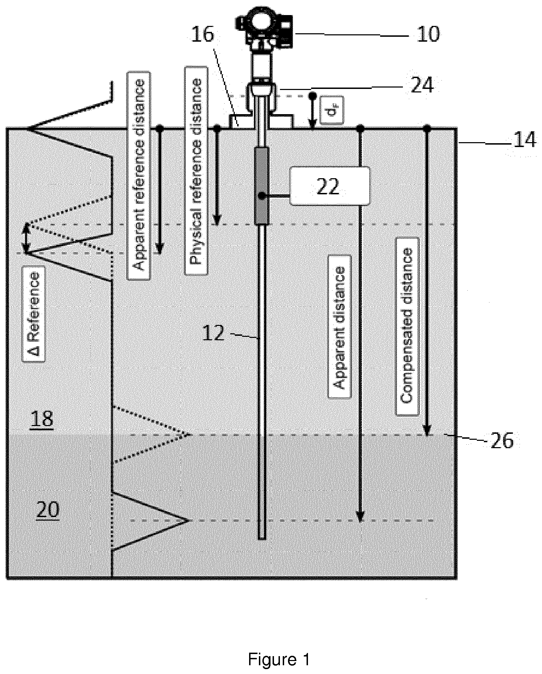

A GWR level sensor is a time-domain reflectometer (TDR) device (time-of-flight measuring device). An example of such a device is show in in Figure 1 and comprises an electronics module 10 including a microwave signal source and a waveguide 12. The device is mounted in process equipment 14, such as a tank or pipeline, such that the electronics module 10 is mounted on an outer surface of the process equipment 14 at a mounting flange 16. The waveguide 12 projects inside the process equipment 14 such that it extends through a gas phase 18 and into a liquid phase 20. In the waveguide configuration shown in Figure 1, a reference feature comprising a reference rod 22 is provided inside the process equipment below the mounting flange 16. A short section 24 of the waveguide 12 extends through the flange outside the process equipment 14, the electronics module 10 being mounted on the end of this short section 24. In normal use, the waveguide is positioned such that its inner end is located below the surface 26 of the liquid phase 20.

In use, a microwave pulse is emitted from the electronics module 10 and travels down the waveguide 12 to the surface of the liquid 26. At the surface 26, a portion of the energy is reflected and this travels back along the waveguide 12 to the electronics module 10 where it is detected. If the velocity $v$ of the microwave pulse is known, and the time $t$ between emitting the pulse and the receiving the reflection is measured, then the distance $d$ to the liquid surface can be calculated using equation 1. From this measured distance the liquid level is then calculated and output by the transmitter.

$$d = \frac{v \cdot t}{2} \qquad (1)$$

In a vacuum an electromagnetic wave will travel at the speed of light $c$ which is related to the vacuum permittivity $\varepsilon_0$ and permeability $\mu_0$ by the relationship shown in equation 2.

$$c = \frac{1}{\sqrt{\varepsilon_0 \mu_0}} \qquad (2)$$

The propagation speed of the microwave pulse used for the level measurement will be affected by the gas phase such that the velocity will no longer be the speed of light but will have a lower velocity $v$. Equation 3 relates the velocity to the material properties by the use of the relative permittivity $\varepsilon_r$ and relative permeability $\mu_r$.

$$v = \frac{1}{\sqrt{\varepsilon_0 \varepsilon_r \mu_0 \mu_r}} \qquad (3)$$

For most transparent materials $\mu_r \approx 1$. Taking this into account and combining equations 2 and 3 it can be stated that the velocity varies with the square root of the relative permittivity.

$$v = \frac{c}{\sqrt{\varepsilon_r}} \qquad (4)$$

If the process always contains the same gas type and runs at a constant gas density then it is relatively easy to take account of the reduced propagation speed within the control system, but there are inherent problems with using this method:

- The delay caused by the gas phase must be known.
- It may be possible to calculate the level correction required using published gas data but this is complex for mixtures of gases.
- During operation changes in the gas mixture, the temperature or the pressure can lead to considerable errors.

One way to consistently compensate for this effect is to directly measure the propagation speed through the gas and continuously compensate for it.

The system of Figure 1 has integrated automatic gas phase compensation. This integrated function provides the possibility to correct the level information regarding temperature and pressure changes, which in turn can be related to the change of the relative permittivity of the gas above the liquid surface.

The principle adopted for automatic gas phase compensation is to present a target, the reference rod 22 positioned at a fixed distance along the waveguide as shown in Figure 1.

The step decrease in diameter of the waveguide 12 due to the reference rod 22 creates a radar echo at a known physical distance, the "Physical reference distance". It can be seen that as the gas pressure is increased, and hence the gas relative dielectric, the radar echoes received from the reference target 22 "Apparent reference distance" and the liquid surface 26 "Apparent distance" are shifted downwards appearing to be at a greater distance. Both echoes are shifted by the same factor, therefore the further the distance the greater the shift seen as is evident from the greater shift seen in the measured distance compared to the reference distance. As the delay through the gas phase is measured directly by the reference section this will correct the measured distance "Compensated distance" regardless of the properties of the gas phase across the full temperature and pressure range. This measurement allows the introduction of a so-called "microfactor" that is related to the speed with which the electromagnetic signal propagates along the waveguide 12. A microfactor of 1 means that the signal propagates with the speed of light. In the case of the system of Figure 1, the gas phase compensation is calculated from the apparent shift ΔReference in the measured distance to the echo from the end of the reference section 22 caused by the slower wave propagation speed:

$$microfactor = \frac{Physical\ reference\ distance}{Apparent\ reference\ distance} \qquad (5)$$

This automatic gas phase compensation method can also be used to measure the pressure-dependent relative permittivity (= dielectric constant) $\varepsilon_r$ of the gas phase 18 since the microfactor is directly related to the relative permittivity:

$$\varepsilon_r = \frac{1}{microfactor^2} \qquad (6)$$

The determination of the relative permittivity $\varepsilon_r$ takes place automatically through the determination of the microfactor value (see equation 5) and the conversion of the microfactor value to the relative permittivity by using equation 6.

The Clausius-Mossotti equation relates the macroscopic property relative dielectric constant $\varepsilon_r$ to the microscopic property polarisability $\alpha$:

$$P_m = \frac{\varepsilon_r - 1}{\varepsilon_r + 2} \cdot \frac{1}{\rho_m} = \frac{N_A \alpha}{3\varepsilon_0} = \frac{4\pi}{3} N_A \cdot \alpha' \qquad (7)$$

The molar polarisation $P_m$ is nearly independent from the molar density $\rho_m$. Many gases show a small but significant deviation from an ideal gas and, therefore, from equation 7. These deviations can be considered by means of virial expansion. The virial coefficients characterise interactions between the particles in the system, which makes it possible to represent the temperature and density dependency of the molar polarisation:

$$P_m = \frac{\varepsilon_r - 1}{\varepsilon_r + 2} \cdot \frac{1}{\rho_m} = A_\varepsilon + B_\varepsilon \cdot \rho_m + C_\varepsilon \cdot \rho_m^2 + ... \qquad (8)$$

$A_\varepsilon = 4\pi \cdot N_A \alpha'/3 = N_A \alpha/3\varepsilon_0$ is the first virial coefficient and $B_\varepsilon$ and $C_\varepsilon$ are the second and third virial coefficients. Equation 8 is valid for non-polar gases. For molecules with a permanent dipole moment $\mu$, an additional term contributes to the low-density expansion of the molar polarisation according to the Debye equation:

$$P_m = \frac{\varepsilon_r - 1}{\varepsilon_r + 2} \cdot \frac{1}{\rho_m} = \frac{N_A}{3\varepsilon_0} \left( \alpha + \frac{\mu^2}{3k_B T} \right) = A_\varepsilon + A_\mu \qquad (9)$$

$A_\varepsilon = N_A \mu^2/3\varepsilon_0 k_B T$ is due to the contribution from the permanent dipole moment, which is negligible when the medium is non-polar. Equation 9 can also be expressed by means of virial coefficients, which results in:

$$P_m = \frac{\varepsilon_r - 1}{\varepsilon_r + 2} \cdot \frac{1}{\rho_m} = A_\varepsilon + A_\mu + B_\varepsilon \cdot \rho_m + C_\varepsilon \cdot \rho_m^2 + ... \qquad (10)$$

Because higher-order terms are difficult to extract from data, an empirical form was chosen to extend the correlation to high densities. The final form of the correlation given by Harvey and Lemmon is as follows:

$$P/\rho_m = P_m = A_\varepsilon + A_\mu^*/T + B_\varepsilon \cdot \rho_m + C \cdot \rho_m^D \qquad (11)$$

In this case the electric polarisation $P$ can be expressed as

$$P = P_m \rho_m = (\varepsilon_r - 1)/(\varepsilon_r + 2).$$

Furthermore $A_\mu^* = N_A \mu^2 / 9\varepsilon_0 k_B = A_\mu T$ should clarify the temperature dependence of the coefficient $A_\mu$. The virial coefficients $A_\varepsilon$, $B_\varepsilon$ and $C$ as empirical parameter (in contrast to the equations 8 and 10) were made temperature dependent as follows:

$$A_\varepsilon = a_0 + a_1 \left( \frac{T}{T_0} - 1 \right) \tag{12a}$$

$$B_\varepsilon = b_0 + b_1 \left( \frac{T_0}{T} - 1 \right) \tag{12b}$$

$$C = c_0 + c_1 \left( \frac{T_0}{T} - 1 \right) \tag{12c}$$

Measurements were performed with (1) nitrogen and (2) methane N55 (research methane with high purity) at pressures between 0.1 MPa and 30/35 MPa.

The methane (pressurised gas cylinder with 20MPa pressure and 50 l volume) had the following composition:

| | |
|---|---|
| - $CH_4$ | $\leq$ 99.9995 Vol.-% |
| - $N_2$ | $\leq$ 2 ppmv |
| - $O_2$ | $\leq$ 0.5 ppmv |
| - $H_2O$ | $\leq$ 2 ppmv |
| - $CO_2$ | $\leq$ 0.1 ppmv |
| - Other hydrocarbons | $\leq$ 0.15 ppmv |

The required parameters for the correlation of the molar polarisation $P_m$ with equation 11 are given by Harvey AH, Lemmon EW, "Method for Estimating the Dielectric Constant of N", International Journal of Thermophysics 2005; 26(1): 31-46. Table 1 is an extract from this table which includes the data for nitrogen and methane.

Table 1: Parameters for the correlation of molar polarisation $P_m$ with equation 10 in accordance with *Harvey and Lemmon* |27|

| Fluid | $a_0$ cm$^3$ mol$^{-1}$ | $a_1$ cm$^3$ mol$^{-1}$ | $A_\mu^*$ cm$^3$ mol$^{-1}$ K | $b_0$ cm$^6$mol$^{-2}$ | $b_1$ cm$^6$mol$^{-2}$ | $c_0$ cm$^6$mol$^{-2}$ | $c_1$ cm$^{3(D+1)}$mol$^{-(D+1)}$ | $D$ |
|---|---|---|---|---|---|---|---|---|
| $N_2$ | 4.3872 | 0.00226 | 0 | 2.2060 | 1.1350 | -169.00 | -35.83 | 2.1 |
| $CH_4$ | 6.5443 | 0.01330 | 0 | 8.4578 | 3.7196 | -352.97 | -100.65 | 2.0 |

A summary of the measurement results for the measurements with nitrogen can be found in Table 2 which shows a comparison of measured values $\varepsilon_{meas}$ with theoretical values $\varepsilon_{theo}$ for pressures in the range between 0.01 MPa to 29.96 MPa and temperatures between 12.8 °C to 20.1 °C. The data for the required molar density $\rho_m$ were obtained from P.J. Linstrom and W.G. Mallard (editors): NIST Chemistry WebBook, NIST Standard Reference Database Number 69, National Institute of Standards and Technology (NIST), 2005 (http://webbook.nist.gov/chemistry) considering the respective pressure and temperature.

Table 2: Comparison of the theoretical dielectric constant $\varepsilon_{\text{theo.}}$ according to *Harvey and Lemmon* |27| with the measured dielectric constant $\varepsilon_{\text{meas.}}$ for nitrogen.

| Temp. °C | Pressure MPa | $\rho_m$ mol m$^{-3}$ | $A_\varepsilon$ | $A_\mu^*$ | $B_\varepsilon$ | $C$ | $P_m$ | $\varepsilon_{\text{theo.}}$ - | Microfactor - | $\varepsilon_{\text{meas.}}$ - |
|---|---|---|---|---|---|---|---|---|---|---|
| 12.8 | 0.01 | 4.2062 | 4.38731 | 0 | 2.15520 | -167.39620 | 0.000018 | 1.00006 | 1.0000 | 1.00000 |
| 13.8 | 2.53 | 1066.8417 | 4.38731 | 0 | 2.15142 | -167.27692 | 0.004683 | 1.01411 | 0.9943 | 1.01150 |
| 13.7 | 5.12 | 2165.7068 | 4.38731 | 0 | 2.15179 | -167.28881 | 0.009510 | 1.02880 | 0.9878 | 1.02485 |
| 15.5 | 10.30 | 4298.6210 | 4.38733 | 0 | 2.14505 | -167.07606 | 0.018886 | 1.05775 | 0.9749 | 1.05216 |
| 17.0 | 15.19 | G178.7653 | 4.38734 | 0 | 2.13950 | -166.90078 | 0.027151 | 1.08373 | 0.9638 | 1.07653 |
| 17.8 | 20.09 | 7903.7870 | 4.38735 | 0 | 2.13656 | -166.80804 | 0.034728 | 1.10793 | 0.9545 | 1.09761 |
| 20.1 | 25.00 | 9385.3959 | 4.38737 | 0 | 2.12821 | -166.54422 | 0.041227 | 1.12900 | 0.9459 | 1.11766 |
| 18.6 | 25.47 | 9582.3729 | 4.38735 | 0 | 2.13364 | -166.71580 | 0.042090 | 1.13182 | 0.9443 | 1.12145 |
| 20.1 | 27.58 | 10132.9289 | 4.38737 | 0 | 2.12821 | -166.54422 | 0.044502 | 1.13972 | 0.9412 | 1.12885 |
| 20.1 | 29.96 | 10781.5066 | 4.38737 | 0 | 2.12821 | -166.54422 | 0.047341 | 1.14908 | 0.9382 | 1.13608 |

**EP 3 296 736 B1**

A compilation of the measurement results regarding the investigations with methane using the same method can be found in Table 3 for pressures in the range between 0.07 MPa to 35.04 MPa and temperatures between 10.5 °C to 20.8 °C.

Table 3: Comparison of the theoretical dielectric constant $\varepsilon_{\text{theo.}}$ according to *Harvey and Lemmon* |27| with the measured dielectric constant $\varepsilon_{\text{meas.}}$ for methane.

| Temp. °C | Pressure MPa | $\rho_m$ mol m$^{-3}$ | $A_\varepsilon$ | $A_\mu^*$ | $B_\varepsilon$ | $C$ | $P_m$ | $\varepsilon_{\text{theo.}}$ - | Microfactor - | $\varepsilon_{\text{meas.}}$ - |
|---|---|---|---|---|---|---|---|---|---|---|
| 10.5 | 0.07 | 29.7240 | 6.54481 | 0 | 8.32011 | -349.24432 | 0.000195 | 1.00058 | 1.0000 | 1.00000 |
| 13.3 | 2.64 | 1169.3912 | 6.54495 | 0 | 8.28510 | -348.29694 | 0.007664 | 1.02317 | 0.9916 | 1.01701 |
| 15.4 | 5.32 | 2462.8558 | 6.54505 | 0 | 8.25929 | -347.59847 | 0.016164 | 1.04929 | 0.9804 | 1.04038 |
| 17.9 | 10.00 | 4946.7632 | 6.54517 | 0 | 8.22905 | -346.78009 | 0.032537 | 1.10089 | 0.9580 | 1.08960 |
| 19.4 | 15.01 | 7687.4424 | 6.54524 | 0 | 8.21115 | -346.29578 | 0.050644 | 1.16004 | 0.9333 | 1.14804 |
| 20.1 | 20.14 | 10168.1649 | 6.54528 | 0 | 8.20286 | -346.07146 | 0.067038 | 1.21556 | 0.9131 | 1.19940 |
| 20.4 | 25.00 | 12039.8379 | 6.54529 | 0 | 8.19932 | -345.97565 | 0.079389 | 1.25871 | 0.8993 | 1.23649 |
| 20.7 | 29.99 | 13533.5633 | 6.54531 | 0 | 8.19579 | -345.88004 | 0.089225 | 1.29390 | 0.8861 | 1.27360 |
| 20.8 | 35.04 | 14741.9288 | 6.54531 | 0 | 8.19461 | -345.84821 | 0.097163 | 1.32286 | 0.8758 | 1.30374 |

The actual temperatures were taken into account for the calculation of the theoretical value of the relative permittivity (dielectric constant) in all cases.

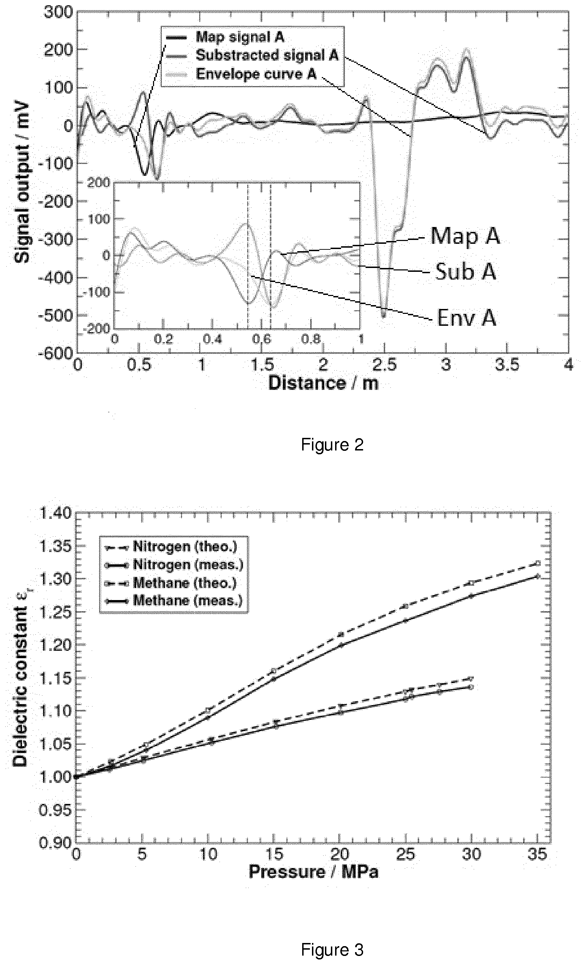

The determination of the microfactor and hence the dielectric constant was performed using the signal data (envelope curves) from the GWR system (see Figure 2 as example for the measurement of methane at 35.04 MPa at a temperature of 20.8 °C). As can be seen the "peak" obtained from the reference position (reference rod) was shifted from 545 mm (map signal A) to 638 mm (envelope curve A). Due to the construction of the system of Figure 1, the dielectric of the gas also affects the signal above the zero reference point of the flange face 16. The correction factor (microfactor) can be calculated according equation 5 with consideration of the additional distance $d_F$ to account for the length of the section 24.

$$microfactor = \frac{Physical\ reference\ distance + d_F}{Apparent\ reference\ distance + d_F} \qquad (13)$$

The value $d_F$ mainly depends on the flange size. For the system of Figure 1 used for these tests, the additional distance has a value of $d_F$ = 96 mm. The result is a microfactor of 0.8733 or a corresponding value for the dielectric constant $\varepsilon_r$ of 1:31122 (see equation 6). A comparison of the values is presented in Figure 3 including measurement data for nitrogen and methane. As can be seen, the measured values have the same characteristic curve as the theoretical values but are below (negative offset) the theoretical curve in both cases.

The bias or systematic error can have many causes. In the case of the system used for the experimental results quoted, the reference rod 22 (500 mm) was installed at a distance of 45 mm from the inner side of the top flange. This results in a physical reference rod distance of 545 mm. Due to the design of the system, the coupling of the GWR signal is already at the fixed distance $d_F$ = 96 mm above the flange. This distance, as recommended value provided by the manufacture of the level meter, is in addition to the design of the level meter mainly based on the thickness of the connection flange 16. As can be seen in the Tables 2 and 3 there is already a deviation between the theoretical and measured dielectric constants at (approximately) atmospheric pressure. These deviations are apparently related to an incorrect $d_F$ value. The determination of a new $d_F$ from the measured values by using a least squares method results in better results. With the calculated value the curve of theoretical dielectric constant and the curve of measured dielectric constant agree within an expanded uncertainty U(k = 2) of 0.5%. Another source of uncertainty is the method itself. The measurement uncertainty can be reduced by using a greater Physical reference distance (distance to the end of the reference rod 22). Any measurement error present in measurement of the reference rod 22 will lead to a measurement error in the measured distance which has to be taken into account. Consequently, the greater the distance to the reference section the smaller the potential error.

GWR devices have been proposed for a number of process monitoring uses. In certain cases, determination of relative permittivity has also been proposed to improve level measurement results. Examples can be found in WO 2016/011531, WO 2004/06663, WO 00/43806, WO 2016/011530, WO 00/437739, GB 2358535, WO 01/18533, US 2009/0303106, and US 2005/0230619.

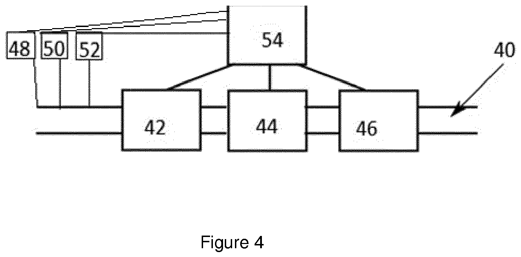

Figure 4 shows a system for measuring the energy content of a gas according to one embodiment of the invention. The system comprises a pipeline 40 through which a gas such as natural gas (NG) flows. A series of sensor systems are mounted on the pipeline 40 to measure the properties of the flowing gas. A first sensor system 42 measures the speed of sound in the gas. This can comprise an ultrasonic flow meter or other speed of sound sensor. The second sensor system 44 comprises a GWR sensor for determining the relative permittivity of the gas. While the GWR systems discussed above comprise level sensors (i.e. they measure the reflections from the liquid surface), it will be clear that as the relative permittivity can be obtained from the reference measurement, a similar sensor could be used in a gas-only environment (i.e. in which there is no liquid surface present). A third sensor system 46 provides the mole fraction of carbon dioxide. This can comprise a $CO_2$ sensor, a non-dispersive infrared (NDIR) sensor, a photoacoustic $CO_2$ sensor, a tuneable laser diode absorption spectroscopy (TLDAS) device, or an on-line infrared gas analyser. Further sensors 48, 50, 52 provide temperature, pressure, and flow rate data. The outputs of the various sensor systems are provided as inputs to an electronics module 54 including data processing capability configures as a correlator to correlate the various measurement in the manner described above to obtain the energy content and energy of the gas. In certain cases, the first sensor system can provide flow rate data, in which case a separate sensor 52 can be omitted.-In certain cases, sensor 52 provides speed-of-sound data, in which case a separate sensor 42 can be omitted.

One variant of this system is in connection with the measurement of the energy content of Liquefied Natural Gas (LNG). LNG has, in contrast to natural gas, a much lower share of nitrogen and carbon dioxide since nitrogen and carbon dioxide have to be removed before the final liquefaction process. Due to the risk of "auto-stratification" the nitrogen content of LNG is kept under 1%. The removal of carbon dioxide from LNG is essential in order to prevent "freeze-out" during the liquefaction process since carbon dioxide would freeze at cryogenic temperatures and could clog the liquefaction equipment such as the heat exchangers. Consequently, the carbon dioxide content is kept to no more than 50 ppm. This means the share of carbon dioxide is therefore negligible for LNG. For the same reason, heavier hydrocarbons are also stripped out so that only methane and some light hydrocarbons remain. Therefore, LNG can be seen in good approximation only as a mixture of light hydrocarbons (C1-C4) and nitrogen, with methane as main share.

Level measurement is a known technology with regard to LNG, especially related to storage tank metering and ship tank level metering where the loaded and unloaded volume of LNG is measured static by means of level differences. By using an appropriate GWR level meter the dielectric constant of the LNG can also be obtained. Because the carbon dioxide content of LNG is so low, its measurement is unnecessary, the measurement of the speed of sound $w$ and the relative permittivity $\varepsilon_r$ being sufficient for all relevant gas properties to be determined by means of correlation methods. For LNG applications, specifically designed ultrasonic flow meters are available for measuring the speed of sound on-line (and real time) under process conditions. The GWR method is particularly suitable for the on-line (and real-time) measurement of relative permittivity $\varepsilon_r$ of LNG. The method of the invention allows the energy content of LNG to be extracted from existing flow and level measurements made during the transportation of LNG, such as during loading and/or offloading of carrier vessels. In addition, the use of measurements from a GWR level sensor can give two separate readings that are affected by $\varepsilon_r$: the echo from the reference rod, and the echo from the liquid surface. While GWR level measurement systems used for LNG typically only consider the microfactor, so as to correct the level measurement, the method of the invention provides a technique in which this "extra" measurement can also be used to obtain $\varepsilon_r$ for use in the correlation to determine energy content.

It will be appreciated that various changes can be made to the embodiments described above while remaining within the scope of the claims.

**Claims**

1. A method of measuring the energy content of a gas, comprising:

   measuring the speed of sound in the gas;
   measuring the relative permittivity of the gas using a guided wave radar sensor; and
   correlating the measured speed of sound and relative permittivity to derive the energy content of the gas.

2. A method as claimed in claim 1, further comprising measuring pressure and temperature parameters of the gas, and using the parameters to derive the energy content of the gas.

3. A method as claimed in claim 1 or 2, wherein the gas is flowing in a conduit, the measurements being made on the flowing gas, optionally further comprising measuring flow rate parameters, and using the flow rate parameters, together with the other measurements, to derive the energy content of the gas.

4. A method as claimed in claim 1 or 2, wherein the gas comprises liquefied gas in a container, the measurement of relative permittivity being derived from a level measurement using the guided wave radar sensor, optionally wherein the speed of sound measurement is made as part of a flow measurement during loading of liquefied gas into the container, or during unloading of liquefied gas from the container.

5. A method as claimed in any preceding claim, wherein measuring the relative permittivity of the gas comprises a time domain reflectometry measurement of a microwave pulse in a wave guide.

6. A method as claimed in any preceding claim, further comprising measuring the carbon dioxide content of the gas, and correlating the measured speed of sound, relative permittivity, and carbon dioxide content to derive the energy content of the gas.

7. A system for measuring the energy content of a gas, comprising:

   a first sensor (42) for measuring the speed of sound in the gas;
   a second sensor (44) for measuring the relative permittivity of the gas using a guided wave radar sensor; and
   an electronics module (54) including data processing capability configured to correlate the measured speed of sound and relative permittivity to derive the energy content of the gas.

8. A system as claimed in claim 7, further comprising sensors for measuring pressure and temperature parameters of the gas, and wherein the electronics module (54) is configured to use the parameters to derive the energy content of the gas.

**9.** A system as claimed in claim 7 or 8, further comprising a conduit housing the sensors and through which the gas flows, optionally further comprising further sensors for measuring volumetric flow parameters, wherein the electronics module (54) is configured to use the volumetric flow parameters and the other measurements to derive the energy content of the gas.

**10.** A system as claimed in claim 7 or 8, wherein the gas comprises liquefied gas in a container, the second sensor comprising a guided wave radar level measurement sensor, optionally wherein the first sensor comprises a flow measurement sensor for use during loading of liquefied gas into the container, or during unloading of liquefied gas from the container.

**11.** A system as claimed in any of claims 7 - 10, wherein the second sensor comprises a microwave source and a waveguide of predetermined configuration, the second sensor being configured to provide a time domain reflectometry measurement of a microwave pulse reflected by a predetermined feature of the waveguide configuration for determining the relative permittivity of the gas, optionally wherein the predetermined feature is the end of a reference rod mounted on the waveguide.

**12.** A system as claimed in any of claims 7 - 11, further comprising a third sensor for measuring the carbon dioxide content of the gas, and wherein the correlator is configured to use the measured speed of sound, relative permittivity, and carbon dioxide content to derive the energy content of the gas, optionally wherein the third sensor comprises an infrared sensor.

**13.** A system as claimed in any of claims 7 - 12, wherein the first sensor comprises an ultrasonic flow meter.

**14.** A system as claimed in any of claims 7 - 13, further comprising a processor configured to receive the output of the correlator as an input, and to output values of superior calorific value, Wobbe index and normal density of the gas.

**Patentansprüche**

**1.** Verfahren zum Messen des Energiegehalts eines Gases, umfassend:

Messen der Schallgeschwindigkeit im Gas;
Messen der relativen Dielektrizitätskonstante des Gases mithilfe eines geführten Radarwellensensors; und
Korrelieren der gemessenen Schallgeschwindigkeit und der relativen Dielektrizitätskonstante, um den Energiegehalt des Gases abzuleiten.

**2.** Verfahren nach Anspruch 1, ferner umfassend Messen von Druck- und Temperaturparametern des Gases und Verwenden der Parameter, um den Energiegehalt des Gases abzuleiten.

**3.** Verfahren nach Anspruch 1 oder 2, wobei das Gas in einer Leitung strömt, die Messungen am strömenden Gas vorgenommen werden, optional ferner umfassend das Messen von Strömungsratenparametern und das Verwenden der Strömungsratenparameter, zusammen mit den anderen Messungen, um den Energiegehalt des Gases abzuleiten.

**4.** Verfahren nach einem der Ansprüche 1 oder 2, wobei das Gas Flüssiggas in einem Behälter umfasst, die Messung der relativen Dielektrizitätskonstante von einer Pegelmessung mithilfe des geführten Radarsensors abgeleitet wird, optional wobei die Messung der Schallgeschwindigkeit als Teil einer Strömungsmessung während des Ladens des flüssigen Gases in den Behälter oder während des Entladens des flüssigen Gases aus dem Behälter ausgeführt wird.

**5.** Verfahren nach einem der vorstehenden Ansprüche, wobei das Messen der relativen Dielektrizitätskonstante des Gases eine Zeitbereichreflektormetriemessung eines Mikrowellenimpulses in einem Wellenleiter umfasst.

**6.** Verfahren nach einem der vorstehenden Ansprüche, ferner umfassend Messen des Kohlendioxidgehalts des Gases und Korrelieren der gemessenen Schallgeschwindigkeit, relativen Dielektrizitätskonstante und des Kohlendioxidgehalts, um den Energiegehalt des Gases abzuleiten.

**7.** System zum Messen des Energiegehalts eines Gases, umfassend:

einen ersten Sensor (42) zum Messen der Schallgeschwindigkeit im Gas;
einen zweiten Sensor (44) zum Messen der relativen Dielektrizitätskonstante des Gases mithilfe eines geführten Radarwellensensors; und
ein elektronisches Modul (54), enthaltend eine Datenverarbeitungsfunktion, die konfiguriert ist, um die gemessene Schallgeschwindigkeit und die relative Dielektrizitätskonstante zu korrelieren, um den Energiegehalt des Gases abzuleiten.

8. System nach Anspruch 7, ferner umfassend Sensoren zum Messen von Druck- und Temperaturparametern des Gases, und wobei das elektronische Modul (54) konfiguriert ist,
um die Parameter zum Ableiten des Energiegehalts des Gases zu verwenden.

9. System nach Anspruch 7 oder 8, ferner umfassend eine Leitung, die die Sensoren beherbergt und durch die das Gas strömt, optional ferner umfassend weitere Sensoren zum Messen volumetrischer Strömungsparameter, wobei das elektronische Modul (54) konfiguriert ist, um die volumetrischen Strömungsparameter und die anderen Messungen zum Ableiten des Energiegehalts des Gases zu verwenden.

10. System nach einem der Ansprüche 7 oder 8, wobei das Gas Flüssiggas in einem Behälter umfasst, der zweite Sensor einen geführten Radarwellensensor für die Pegelmessung umfasst, optional wobei der erste Sensor einen Strömungsmessungssensor zur Verwendung während des Ladens des flüssigen Gases in den Behälter oder während des Entladens des flüssigen Gases aus dem Behälter umfasst.

11. System nach einem der Ansprüche 7 bis 10, wobei der zweite Sensor eine Mikrowellenquelle und einen Wellenleiter vorgegebener Konfiguration umfasst, wobei der zweite Sensor konfiguriert ist, um eine Zeitbereichsreflektormetriemessung eines durch ein vorgegebenes Merkmal der Wellenleiterkonfiguration reflektierten Mikrowellenimpulses bereitzustellen, um die relative Dielektrizitätskonstante des Gases zu bestimmen, optional wobei das vorgegebene Merkmal das Ende einer am Wellenleiter montierten Referenzstange ist.

12. System nach einem der Ansprüche 7 bis 11, ferner umfassend einen dritten Sensor zum Messen des Kohlendioxidgehalts des Gases, und wobei der Korrelator konfiguriert ist, um die gemessene Schallgeschwindigkeit, relative Dielektrizitätskonstante und den Kohlendioxidgehalt zu verwenden, um den Energiegehalt des Gases abzuleiten, optional wobei der dritte Sensor einen Infrarotsensor umfasst.

13. System nach einem der Ansprüche 7 bis 12, wobei der erste Sensor einen Ultraschallströmungsmesser umfasst.

14. System nach einem der Ansprüche 7 bis 13, ferner umfassend einen Prozessor, der konfiguriert ist, um den Ausgang des Korrelators als Eingang zu empfangen und Werte des Brennwerts, Wobbe-Indexes und der Normaldichte des Gases auszugeben.

**Revendications**

1. Procédé de mesure de la teneur énergétique d'un gaz, comprenant :

la mesure de la vitesse du son dans le gaz ;
la mesure de la permittivité relative du gaz à l'aide d'un capteur radar à ondes guidées ; et
la corrélation de la vitesse du son et de la permittivité relative mesurées pour en déduire la teneur énergétique du gaz.

2. Procédé selon la revendication 1, comprenant en outre la mesure des paramètres de pression et de température du gaz et l'utilisation des paramètres pour déduire la teneur énergétique du gaz.

3. Procédé selon la revendication 1 ou 2, ledit gaz circule dans un conduit, lesdites mesures étant effectuées sur le gaz en écoulement, éventuellement comprenant en outre la mesure de paramètres de débit et l'utilisation des paramètres de débit ensemble avec les autres mesures pour déduire la teneur énergétique du gaz.

4. Procédé selon la revendication 1 ou 2, ledit gaz comprenant un gaz liquéfié dans un récipient, ladite mesure de la permittivité relative étant déduite d'une mesure de niveau à l'aide du capteur radar à ondes guidées, éventuellement, ladite mesure de la vitesse du son étant effectuée dans le cadre d'une mesure de débit durant le chargement du gaz liquéfié dans le récipient ou lors du déchargement du gaz liquéfié du récipient.

5. Procédé selon l'une quelconque des revendications précédentes, ladite mesure de la permittivité relative du gaz comprenant la mesure de réflectométrie dans le domaine temporel d'une impulsion micro-onde dans un guide d'onde.

6. Procédé selon l'une quelconque des revendications précédentes, comprenant en outre la mesure de la teneur en dioxyde de carbone du gaz et la corrélation de la vitesse du son, de la permittivité relative et de la teneur en dioxyde de carbone mesurées pour déduire la teneur énergétique du gaz.

7. Système destiné à la mesure de la teneur énergétique d'un gaz, comprenant :

   un premier capteur (42) destiné à mesurer la vitesse du son dans le gaz ; un deuxième capteur (44) destiné à mesurer la permittivité relative du gaz
   à l'aide d'un capteur radar à ondes guidées ; et
   un module électronique (54) comprenant une capacité de traitement de données conçue pour corréler la vitesse du son et la permittivité relative mesurées pour déduire la teneur énergétique du gaz.

8. Système selon la revendication 7, comprenant en outre des capteurs destinés à mesurer des paramètre de pression et de température du gaz,
   et ledit module électronique (54) étant conçu pour utiliser
   les paramètres pour déduire la teneur énergétique du gaz.

9. Système selon la revendication 7 ou 8, comprenant en outre un conduit logeant les capteurs et à travers lequel le gaz s'écoule, éventuellement, comprenant en outre des capteurs supplémentaires destinés à mesurer des paramètres de débit volumétrique, ledit module électronique (54) étant conçu pour utiliser les paramètres de débit volumétrique et les autres mesures pour déduire la teneur énergétique du gaz.

10. Système selon la revendication 7 ou 8, ledit gaz comprenant du gaz liquéfié dans un récipient, ledit deuxième capteur comprenant un capteur de mesure de niveau radar à ondes guidées, éventuellement, ledit premier capteur comprenant un capteur de mesure d'écoulement destiné à être utilisé durant le chargement de gaz liquéfié dans le récipient ou durant le déchargement du gaz liquéfié du récipient.

11. Système selon l'une quelconque des revendications 7 à 10, ledit deuxième capteur comprenant une source de micro-ondes et un guide d'ondes de configuration prédéfinie, ledit deuxième capteur étant conçu pour fournir une mesure de réflectométrie dans le domaine temporel d'une impulsion micro-onde réfléchie par une caractéristique prédéfinie de la configuration de guide d'ondes en vue de la détermination de la permittivité relative du gaz, éventuellement ladite caractéristique prédéfinie étant l'extrémité d'une tige de référence montée sur le guide d'ondes.

12. Système selon l'une quelconque des revendications 7 à 11, comprenant en outre un troisième capteur destiné à mesurer la teneur en dioxyde de carbone du gaz, et ledit corrélateur étant conçu pour utiliser la vitesse du son mesurée, la permittivité relative et la teneur en dioxyde de carbone pour déduire la teneur énergétique du gaz, éventuellement ledit troisième capteur comprenant un capteur infrarouge.

13. Système selon l'une quelconque des revendications 7 à 12, ledit premier capteur comprenant un débitmètre à ultrasons.

14. Système selon l'une quelconque des revendications 7 à 13, comprenant en outre un processeur configuré pour recevoir la sortie du corrélateur en tant qu'entrée et pour délivrer en sortie des valeurs de valeur calorifique supérieure, d'indice de Wobbe et de densité normale du gaz.

Figure 1

Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6244097 B
- US 627380 A
- US 20020124630 A
- WO 2016011531 A
- WO 200406663 A
- WO 0043806 A
- WO 2016011530 A
- WO 00437739 A
- GB 2358535 A
- WO 0118533 A
- US 20090303106 A
- US 20050230619 A

**Non-patent literature cited in the description**

- **M. JAESCHKE.** Thermodynamic research improves energy measurement of natural gas. *Thermochimica Acta,* 2002, vol. 382, 37-45
- **HARVEY AH ; LEMMON EW.** Method for Estimating the Dielectric Constant of N. *International Journal of Thermophysics,* 2005, vol. 26 (1), 31-46
- NIST Standard Reference Database Number 69. NIST Chemistry WebBook. National Institute of Standards and Technology (NIST), 2005